# EUROPÄISCHE PATENTSCHRIFT

(11) **EP 0 463 006 B1**
(45) Veröffentlichungstag und Bekanntmachung des Hinweises auf die Patenterteilung: **18.08.1993**
(21) Anmeldenummer: 90904229.3
(22) Anmeldetag: 14.03.1990
(51) Int. Cl.: A61F 2/46, A61B 17/22, A61B 19/00

(54) **VORRICHTUNG ZUM ENTFERNEN EINER KNOCHENZEMENTRÖHRE**
DEVICE FOR REMOVING A BONE CEMENT TUBE
PROCEDE POUR ENLEVER DES TUBES DE CIMENTATION DES OS

(30) Priorität: 14.03.1989 DE 8903310 U; 09.10.1989 DE 3933711
(43) Veröffentlichungstag der Anmeldung: 02.01.1992
(73) Patentinhaber: ZAHEDI, Amir, D-48143 Münster (DE)
(72) Erfinder: ZAHEDI, Amir, D-48143 Münster (DE)
(74) Vertreter: Habbel, Hans-Georg, Dipl.-Ing.
(86) Internationale Anmeldenummer: DE9000200
(87) Internationale Veröffentlichungsnummer: WO9010423

(56) Entgegenhaltungen:
- EP-A- 0 028 712
- EP-A- 0 305 627
- WO-A-87/02571
- DE-A- 2 741 107

## Beschreibung

Die Erfindung betrifft eine Vorrichtung der im Oberbegriff des Anspruchs 1 angegebenen Art.

Einzementierte Implantate können im allgemeinen nicht beliebig lange im Knochen verbleiben. Falls ein solches Implantat ausgewechselt oder herausgenommen werden muß, verbleibt nach dessen Entfernung eine im wesentlichen zylindrische Knochenzementröhre im Knochen, die zur Reimplantation vollständig entfernt werden muß.

Das Entfernen des alten Knochenzementes erfolgte bisher durch Ausbohren von oben oder durch Bypaßlegung schräg von unten, um alle Knochenzementteile lösen zu können und mittels Zangen bzw. sonstiger mechanischer Hilfsmittel, insbesondere Absaugvorrichtungen.

Es ist weiterhin bekannt, sich zu diesem Zweck eines Extraktors zu bedienen, der an seinem Kopf ein Pfropfengewinde aufweist, welches in die Knochenzementröhre eingeschnitten wird, um auf diese Art und Weise genügend Halt zum Austreiben der Knochenzementröhre zu erhalten. Dieses bekannte Instrument hat den erheblichen Nachteil, daß insbesondere bei älteren Patienten die Gefahr besteht, daß die Knochenwandung aufsplittert, wenn die durch das Pfropfengewinde erzeugte Radialspannung größer ist als die Festigkeit der Knochen. Auch ist bei Verwendung eines Extraktors nicht sichergestellt, daß im Fußpunkt der im Knochen befindlichen Höhlung sämtlicher Knochenzement abgelöst wird.

Aus der DE-A-29 44 710 ist ferner eine Kombination eines derartigen Extraktors mit einem Spreizinstrument bekannt, wobei das Spreizinstrument die Aufgabe hat, die Knochenzementröhre zu untergreifen, um somit das Austreiben der Zementröhre zu erleichtern. Aber auch die Anwendung dieser Vorrichtung ist mit erheblichen mechanischen Belastungen der die Knochenzementröhre umgebenden Kortikalis verbunden. Die oben erwähnten Unsicherheiten in Bezug auf die Vollständigkeit der Zemententfernung werden durch das Spreizinstrument nicht beseitigt. Außerdem kann es zum Untergreifen der Knochenzementröhre notwendig sein, die vorhandene Knochenhöhlung zu vertiefen, um entsprechenden Arbeitsraum für das Spreizinstrument unterhalb der Knochenzementröhre zu gewinnen. Nachteilig ist darüberhinaus, daß eine optische Überwachung dieser Manipulation und des Einsatzes des Spreizinstrumentes, beispielsweise über ein auf einen Monitor übertragenes Röntgenbild, erforderlich ist.

Aus der DE-A-27 41 107 ist ein durch Ultraschall erregtes und in die Knochenhöhlung nach Entfernung der Prothese einführbares Werkzeug bekannt, mit dem die Knochenzementröhre vom Knochen lösbar ist. Nachteilig dabei ist, daß die Ultraschall abstrahlende Sonotrode lediglich mit dem oberen Ende des Werkzeugs in Verbindung steht, so daß die erzeugbare Schwingungsenergie am unteren Ende eines längeren Werkzeugs deutlich niedriger ist als im oberen Bereich . Dadurch wird gerade der untere Teil der Knochenzementröhre, der aufgrund der sich oft verjüngenden Prothesenschäften einen größere Wandstärke aufweist, nicht ausreichend gelockert.

Der Erfindung liegt die Aufgabe zugrunde eine Vorrichtung der eingangs genannten Gattung derart zu verbessern, daß die Auslockerung einer Knochenzementröhre und ihre Herausnahme aus der Knochenhöhlung in einem Stück oder in wenigen Bruchstücken gestattet.

Diese Aufgabe wird mit den kennzeichnenden Merkmalen des Anspruchs 1 gelöst.

Die Erfindung beruht auf der Erkenntnis, daß ein Ultraschallerzeuger, der einen in den Hohlraum der Knochenzementröhre einführbaren Führungsabschnitt aufweist, dessen freies Ende die ultraschallemittierende Sonotrode bildet in die Knochenzementröhre, aus der der Prothesenschaft entfernt wurde, einführbar ist und die Sonotrode damit in die Nachbarsehaft aller Bereiche der durch die Knochenröhre gebildeten Wandung gelangen kann. Die Querabmessungen der Sonotrode sind dabei jeweils kleiner als diejenigen der entsprechenden Bereiche der Knochenzementröhre bzw. des zugeordneten Prothesenschafts, so daß er sich vollständig in die Zementröhre einführen läßt, und die Länge ist der des Innenraums der Knochenzementröhre bzw. der Länge des Prothesenschafts angepaßt. Dadurch, daß die Sonotrode das freie Ende des Führungsabschnitts bildet, können sämtliche Teile der Röhre durch ein langsames Ein- bzw. Ausfahren des Führungsabschnitts behandelt werden. Bei dem "Führungsabschnitt" handelt es sich also um einen in die Knochenröhre einführbaren Teil, der somit die "Führung" innerhalb der Knochenröhre ganz oder teilweise übernimmt, so daß sich eine unkomplizierte Handhabung ergibt. Die ultraschallemittierende Sonotrode befindet sich im Endbereich des Führungsabschnitts und gelangt somit in den tiefsten Teil der Knochenröhre, wenn der Führungabschnitt vollständig eingeführt ist. Auf dem Einführungsweg oder bei Wiederhinausführen können alle anderen Teile der Knochenröhre erreicht werden.

Insbesondere weist das so gebildete Handgerät den genannten Führungsabschnitt auf, der in die Knochenhöhlung bis zu einem Anschlag einschiebbar ist. Auf diese Weise läßt sich der Ultraschallerzeuger in dem nach Entfernen des Prothesenschaftes offenstehenden Hohlraum in axialer Richtung führen, so daß dar Ultraschallerzeugern nacheinander in die Nachbarschaft aller Bereiche der Wandung der im Knochen verbleibenden Zementröhre gelangt.

Es wurde gefunden, daß eine auf Knochensubstanz aufgebrachte Zementschicht bei Bestrahlung mit Ultraschall mit einer Frequenz im Bereich von 40 kHz sicher auslockert und als kompletter Zementblock von der Knochenoberfläche abnehmbar ist. Die Knochensubstanz wird dabei nicht angegriffen.

Ein derartiger Ultraschallerzeuger besteht insbesondere aus zwei Teilen - einem Handgerät und einem über ein flexibles Hochfrequenzkabel angeschlossenen Generator. Das Handgerät weist neben einem außerhalb des Knochens verbleibenden Griffteil einen zum Führen innerhalb der Knochenröhre bestimmten Einführungsabschnitt auf, der mit einer ultraschallemittierenden Sonotrode an seinem freien Ende versehen ist.

Die Sonotrode weist als Endbereich des Führungsabschnitts bevorzugt eine abgerundete Form auf, so daß in jeder Position ein kufenartiges Gleiten in der Zementröhre möglich ist und ein Sperren der Gleitbewegung durch Verkanten des Führungsabschnitts verhindert ist.

Insbesondere wird die aktive Fläche der Sonotrode durch die Mantelfläche eines koaxial zur Längsachse des Führungsabschnittes ausgerichteten im wesentlichen zylindrischen Bereichs gebildet. Damit besteht die Sonotrode mit dem Führungsabschnitt aus einem homogenen Körper, vorzugsweise Titan oder Aluminium, in den vom außerhalb der Knochenröhre befindlichen Teil mittels eines Schwingers oder dergleichen die Ultraschallenergie eingekoppelt wird. Cie Abstrahlung erfolgt dann in radialer Richtung über die äußere Oberfläche der in den Führungsabschnitt integrierten Sonotrode. Dabei ist bevorzugt jeweils der Flächenvektor der aktiven Fläche der Sonotrode radial zur Längsachse des Führungsabschnittes gerichtet.

Zur Ausbildung einer stehenden Welle sind die Länge der Sonotrode und die Ultraschallfrequenz derart bemessen, daß die Länge der Sonotrode im wesentlichen gleich einem Vielfachen der halben sich innerhalb der Sonotrode einstellenden Wellenlänge ist.

Entsprechend einer vorteilhaften Weiterbildung der Erfindung weist der sich zu seinem freien Ende hin verjüngende die Sonotrode enthaltende Führungsabschnitt einen dem Prothesenschaft angepaßten gekrümmten Bereich auf, wobei die Krümmung jeweils so gewählt ist, daß der einen konstanten querschnitt aufweisende oder sich zu seinem freien Ende hin gleichmäßig verjüngende Führungsabschnitt bei insgesamt möglichst geringer Krümmung des Sonotrodenbereichs den am weitesten von dem äußeren Zugang entfernten Bereich der Knochenröhre erreicht. Auf diese Weise wird auch der von der Zugangsöffnung am weitesten entfernt liegende Bereich der Knochenröhre bei möglichst gerader Ausbildung des Führungsabschnitts erreicht. Durch die gerade Ausführung des Sonotrodenbereichs werden Biegeschwingungen, welche die Sonotrode schädigen können möglichst vermieden bzw. gering gehalten.

Um eine Anwendung im Zusammenhang mit möglichst vielen unterschiedlich geformten Prothesenschäften zu ermöglichen, sind mehrere auswechselbare Sonotroden aufweisende Führungsabschnitte vorgesehen.

Entsprechend einer anderen vorteilhaften Weiterbildung der Erfindung ist zur Aufnahme und Arretierung des Führungsabschnittes des Ultraschallerzeugers ein Führungsrohr vorgesehen, das sich in das Innere der Zementröhre einschieben läßt. Das Führungsrohr ermöglicht ein gleichmäßiges Verschieben der Sonotrode, so daß die gesamte Grenzfläche zwischen der Knochenzementröhre und dem Knochen von den Ultraschall überstrichen wird. Emittiert die Sonotrode nicht konzentrisch um die Spitze des Führungsabschnittes sondern in einer Vorzugsrichtung radial zum Führungsrohr, ergibt sich damit eine komplizierte mäanderartige Bewegung. Die Sonotrode wird um jeweils 360° abzüglich des radialen Öffnungswinkels der emittierten Ultraschall hin- und hergedreht und dabei gleichzeitig axial verschoben. Diese Bewegungsart ist einer fortlaufenden Drehbewegung mit überlagerter Verschiebung vorzuziehen, da hierbei das Handgerät des Ultraschallerzeugers nicht mitsamt dem Hochfrequenzkabel verdreht zu werden braucht. Ein derartiger Bewegungsablauf läßt sich auf einfache Weise mittels eines entsprechenden Topfkurvenzylinders realisieren, der entweder auf den Rand der Knochenzementröhre extern aufgesteckt wird und in deren mäanderartiger Ausnehmung ein Nippel des Führungsrohres eingreift oder die in das Führungsrohr direkt eingearbeitet ist und als "Führungsschiene" für einen Nippel des Führungsabschnïttes des Ultraschallerzeugers dient. In dieser Ausführungsform behält das Führungsrohr seine Relativlage zur Knochenzementröhre bei und der Führungsabschnitt wird gleichsam "herausgewunden", während im ersten Fall der Führungsabschnitt zum Führungsrohr arretiert ist und beide Teile zusammen aus der Röhre herausgewunden werden.

Vorteilhaft ist weiterhin, wenn ein derartiges Führungsrohr zum Durchlaß eines Endoskopes, einer Spülvorrichtung und/oder eines Spreizinstrumentes ausgebildet ist.

Das Führungsrohr kann, entsprechend einer Weiterbildung der Erfindung darüberhinaus auch im Austausch mit dem Führungsabschnitt zur Aufnahme eines Endoskopes und/oder einer Spülvorrichtung und/oder eines Spreizinstrumentes genutzt werden. Spreizinstrumente ermöglichen durch ein Untergreifen der Knochenzementröhre ein bequemes Herausheben der gelockerten Zementröhre, auch wenn nach der Ultraschallbehandlung noch ein geringes Nachlockern mittels Hammer und Meißel erforderlich war und die Zementröhre dabei möglicherweise in mehrere Teile zerbrochen ist.

Gemäß einer vorteilhaften Weiterbildung der Erfindung ist die Sonotrode der Form des entfernten Prothesenschaftes und damit der Höhlung der Zementröhre näherungsweise angepaßt. Die Maße einer derartigen Sonotrode können demzufolge den Maßen der für die Aushöhlung des Knochens verwendeten Raspel entlehnt sein. Die Länge der Sonotrode kann die der Zementröhre übertreffen, während der Durchmesser maximal dem der Zementröhre entspricht und zur Spitze abnimmt. Eine geringe säbelförmige Krümmung erleichtert das Einsetzen der Sonotrode in die Knochenzementröhre. Durch die Ausbildung der gesamten äußeren Sonotrodenoberfläche als ultraschallemittierende Fläche ist die Verwendung einer Führungshilfe, insbesondere eines Führungsrohres oder eines Topfkurvenzylinders, nicht erforderlich. Die Zahl der Varianten dieser speziellen, der Knochenzementröhre angepaßten Sonotrodenform richtet sich nach den üblichen Schaftlängen und den Krümmungsradien der proximalen Schaftenden.

Weitere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 in einem Übersichtsschema verschiedene Baueinheiten und deren Kombinierbarkeit,
Figur 2 ein weiteres Ausführungsbeispiel mit einer in einer Vorzugsrichtung emittierenden Sonotrode in teilweise längsgeschnittener Darstellung,
Figur 3 ein weiteres Ausführungsbeispiel einer in jede radiale Richtung emittierenden Sonotrode in einer Seitenansicht und
Figur 4 eine Übersichtsdarstellung verschiedener Varianten des Ausführungsbeispiels gemäß Figur 3.

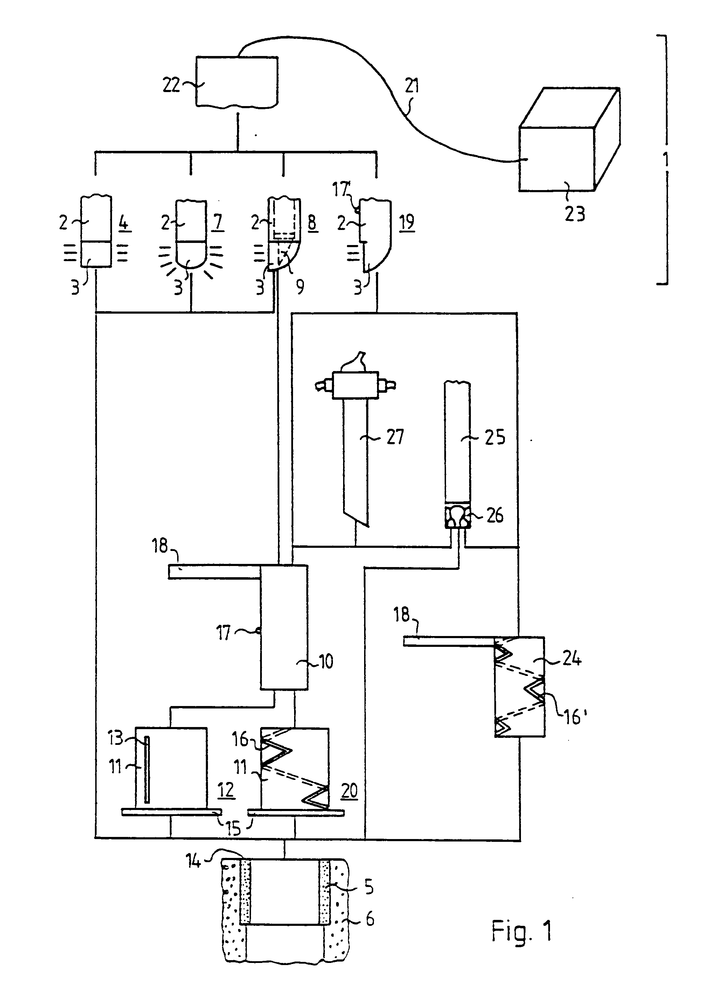

Die zentrale Baueinheit des in Figur 1 dargestellten Ausführungsbeispiels der erfindungsgemäßen Vorrichtung zum Entfernen einer Knochenzementröhre bei einer Reimplantation einer Endoprothese aus einer Knochenhöhlung wird durch einen Ultraschallerzeuger 1, der einem zylinderförmigen Führungsabschnitt 2 aufweist, gebildet. Der einen Schaft bildende Führungsabschnitt 2 ist an seinem freien Ende mit einer ultraschallemittierenden Sonotrode 3 versehen. An seinem nicht dargestellten oberen Ende befindet sich ein Handgriff zum Führen des Schafts.

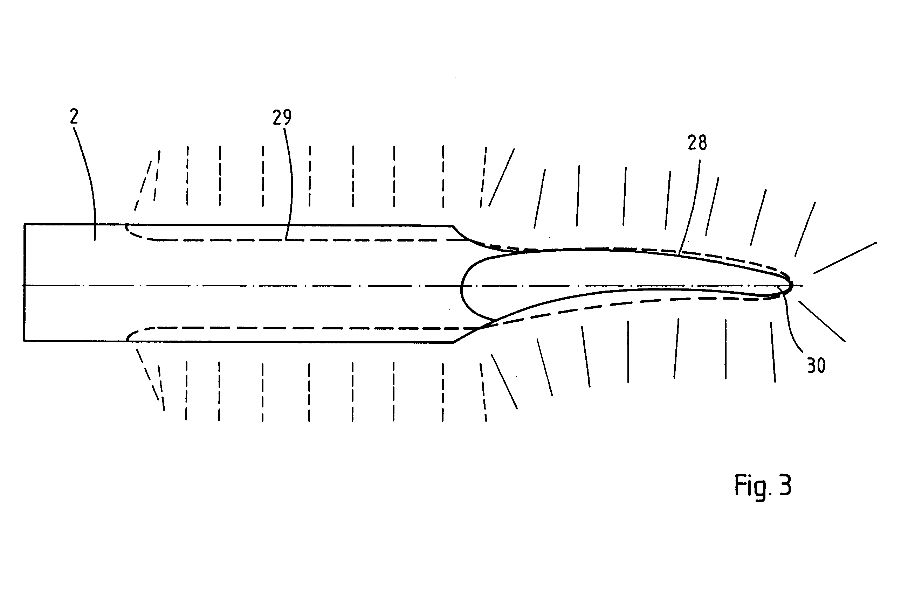

In Figur 1 sind vier spezielle Gestaltungsformen von Sonotroden dargestellt, die bei diesen Ausführungsformen mehr oder weniger punktförmige Ultraschallquellen bilden. Diese Sonotroden können bei anderen Ausführungsformen - wie sie beispielsweise in Figur 3 dargestellt sind - auch einen mehr oder weniger großen Längenbereich des schaftförmigen Fügrungsabschnitts einnehmen. der Führungsabschnitt gestattet es, den Sonotrodenbereich in den von dem Prothesenschaft nach dessen Entfernen freigegebenen Hohlraum einzuführen.

Bei einer ersten Gestaltungsform 4 werden von einer zylindermantelartigen Sonotrodenoberfläche Ultraschall ausgesandt. Bei einer derartigen Gestaltungsform 4 wird eine gleichmäßige Rundumbestrahlung einer inneren Oberfläche eines Hohlzylinders erreicht, ohne die Sonotrode 3 drehen zu müssen. Ein Führungsabschnitt 2 eines Ultraschallerzeugers 1 mit einer Sonotrode der Form 4 kann also direkt in eine Knochenzementröhre 5 hineingesteckt werden um dann unter Absendung von Ultraschall wieder herausgezogen zu werden. Auf diese Weise wird jedes Flächenelement der Grenzfläche zwischen der Knochenzementröhre 5 und dem Knochen 6 von den Ultraschall erreicht. Die gleiche Arbeitsweise wird auch bei einer mit 7 bezeichneten zweiten Sonotrode praktiziert, die die Form einer Halbkugel hat und an der Spitze des Führungsabschnittes 2 angeordnet ist.

Sendet die Sonotrode dagegen nur in axialer Richtung des Führungsrohres Ultraschall aus - wie das dritte Ausführungs beispiel 8 zeigt, so müssen die Wellen mit Hilfe eines entsprechenden optischen Elementes 9 seitlich umgelenkt werden. Vorteilhafterweise ist das optische Element 9 manuell oder motorisch drehbar, so daß auch hier wieder eine gleichmäßige Rundumbestrahlung resultiert. Damit kann ein derartiger Ultraschallerzeuger 1 direkt in die Zementröhre 5 hineingesteckt werden und wird von dieser manuell geführt.

Falls eine kontrollierte Zwangsführung erwünscht ist, ist diese ebenfalls in günstiger Weise realisierbar: Der Führungsabschnitt 2 mit der Sonotrode 3 wird in ein Führungsrohr 10 eingesteckt, so daß dieses mittels eines hohlen Topfkurvenzylinders 11 entlang einer Ausnehmung gleitet, deren Form eine Bewegung der Sonotrode zum Überstreichen der gesamten Röhrenoberfläche ermöglicht. Im einfachsten Fall ist bei einer ersten Gestaltungsform 12 des Topfkurvenzylinders 11 dieser lediglich mit einem Schlitz 13 in axialer Richtung versehen. Der Topfkurvenzylinder 11 der Gestaltungsform 12 wird auf den Rand 14 der Knochenzementröhre 5 aufgesetzt und ist deshalb mit einem schmalen Kragen 15 versehen. Zum Eingreifen in die der Kurve der gewünschten Relativbewegungen von Sonotrobe 3 und Knochenzementröhre 5 entsprechenden länglichen Ausnehmung 16 bei einer anderen Gestaltungsform 20 eines Topfkurvenzylinders 11 besitzt das Führungsrohr 10 einen vorstehenden Nippel 17. Dieser Nippel 17 ist bevorzugt durch leichte Kraftwirkung gegen eine kleine Feder, die in einer Vertiefung in der Wandung des Führungsrohres 10 angeordnet ist, in diese Vertiefung versenkbar. Dadurch wird das Einschnappen des Nippels 17 in die Ausnehmung 16 erleichtert. Zum manuellen Führen entlang der Form des Schlitzes ist das Führungsrohr 10 mit einem Griff 18 ausgestattet.

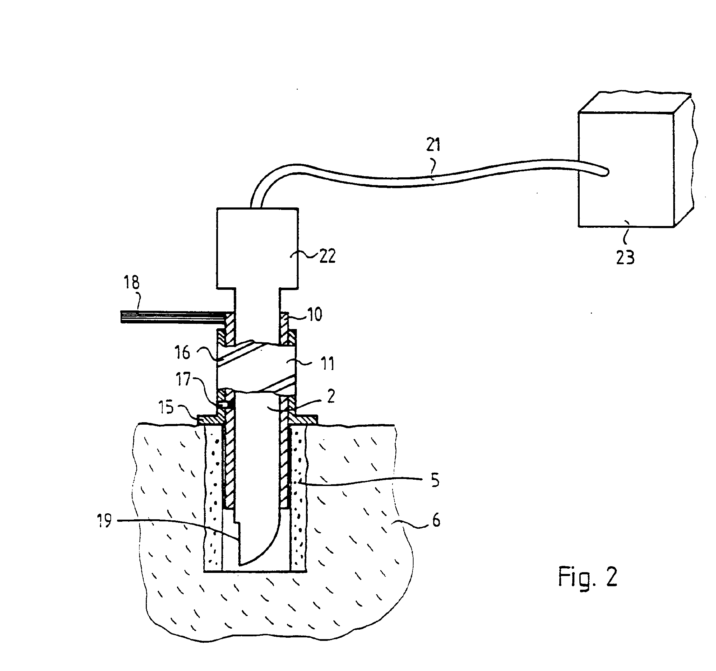

Die Ausnehmung 16 ist komplizierter gestaltet, wenn die Sonotrode 3 Ultraschall nicht in alle radialen Richtungen aussendet, sondern nur innerhalb eines bestimmten Winkelbereiches. Das ist bei der vierten Gestaltungsform -mit 19 beziffert - der Sonotrode 3 der Fall. Ein mit einer derartigen Sonotrode ausgerüsteter Führungsabschnitt 2 eines Ultraschallerzeugers 1 muß innerhalb der Knochenzementröhre 5 so geführt werden, daß alle Flächenelemente der Grenzfläche zwischen der zu entfernenden Knochenzementröhre 5 und der umgebenden Knochensubstanz 6 mit der gleichen Intensität bestrahlt werden. Das ist vorteilhaft dadurch realisierbar, daß der Führungsabschnitt 2 in ein Führungsrohr 10 eingeschoben und arretiert wird und dieses anschließend mit seinem Nippel 17 in die gewundene Ausnehmung 16 des Topfkurvenzylinders 11 der Gestaltungsform 20 eingerastet und als komplette Einheit in die Knochenzementröhre 5 eingesetzt wird, wobei der Topfkurvenzylinder 11 der Gestaltungsform 20 auf dem Rand 14 der Röhre 5 aufliegt. Die derart ineinandergeschobenen Teile sind in Figur 2 dargestellt. Die Länge des Führungsrohres 10 muß mindestens der Summe der Länge des Topfkurvenzylinders 11 der Gestaltungsform 20 und der Tiefe der Knochenzementröhre 5 entsprechen. Die Form der Ausnehmung 16 kann schraubenlinienförmig oder mäanderförmig sein. Letzteres ist im allgemeinen vorzuziehen, da ein mäanderartiges Hin- und Herdrehen gegenüber einem schraubenartigen vollständigen Drehen den Vorteil hat, daß das Hochfrequenzkabel 21, welches das Handgerät 22 mit dem Generator 23 verbindet, nicht verdrillt wird.

Die gleiche Ausnehmung 16' kann auch direkt in ein entsprechendes Führungsrohr 24 eingeschnitten sein. Dann muß der Führungsabschnitt 2 den Nippel 17' aufweisen. Bei dieser Variante wird das Handgerät 22 des Ultraschallerzeugers 1 geführt. Die wandung des Führungsrohres 24, die in diesem Falle noch zwischen der Sonotrode 3 und der Knochenzementröhre 5 befindlich ist, stellt kein Hindernis für die Ultraschall dar.

Nach dem Lockern der Knochenzementröhre 5 wird diese vorzugsweise mit Hilfe eines Spreizinstrumentes 25 komplett herausgenommen. Das Spreizinstrument 25 ist an seinem distalen Ende mit aufspreizbaren Lamellen 26 versehen. Diese Lamellen untergreifen die Zementröhre 5 bzw. verklemmen sich innerhalb der Röhre 5. Soll jedoch ein Austausch des Führungsrohres mit einer endoskopischen Einrichtung 27 möglich sein, muß aufgrund der erforderlichen Sichtfreiheit ein oben erläutertes Führungsrohr 10 mit einem Topfkurvenzylinder 11 eingesetzt werden. Das Endoskop 27 dient der Durchmusterung des Hohlraumes insbesondere nach dem Herausnehmen der Knochenzementröhre 5 sowie dem Spülen und dem Absaugen kleinerer Partikel.

Figur 3 zeigt zwei Varianten 28 und 29 - durchgezogene und gestrichelte Kontur - einer weiteren Ausführungsform einer zur manuellen Führung bestimmten Sonotrode mit Führungsabschnitt, die radial zu ihrer gesamten Oberfläche Ultraschall emittiert. Die Länge der Sonotrode entspricht mindestens der der auszulockernden Zementröhre und die Dicke höchstens der der Zementröhre, wobei die Dicke zur Sonotrodenspitze 30 hin abnimmt. Die Spitze 30 ist sphärisch verrundet ausgebildet. Die Sonotrode 28 bzw. 29 weist eine geringfügige säbelartige Krümmung auf, deren sich in Richtung der Längsachse stetig ändernder Krümmungsradius an jeder Stelle ein Mittelwert der zugeordneten Stelle aller Schaftkrümmungen einer Klasse gleichartiger Endoprothesen, d.h. der Endoprothesen annähernd gleicher Länge und ähnlicher Krümmungsradien, darstellt. Eine auf diese Weise formmäßig optimierte Sonotrode gestattet beispielsweise die Entfernung von innerhalb eines Variationsbereiches unterschiedlich geformten Knochenzementröhren von entfernten entsprechenden Schaftprothesen annähernd gleicher Länge. Der schwingfähige ultraschallabstrahlende Sonotrodenteil kann sich dabei vom freien Ende her - je nach gewünschter Ausführung - mehr oder weniger weit zum Griffende hin erstrecken. Bei der manuellen Führung gleitet die erfindungsgemäße Vorrichtung mit ihrem entsprechend bemessenen Führungsabschnitt in der nach dem Entfernen der Prothese verbleibenden Zementröhre. Auch wenn lediglich der Spitzenbereich schallabstrahlend ist, wird beim drehenden Ein- und Ausführen der Sonotrode mit Führungsabschnitt der gesamte Bereich der Zementröhre erfaßt, so daß sich dieser nach diesem Auslockern leicht entfernen läßt.

Ein gewisses Problem stellt die Krümmung der Schaftprothesen dar, weil der Führungsabschnitt mit Sonotrode dieser Krümmung folgen muß. Andererseits führt eine gekrümmter Sonotrodenbereich zu Biegeschwingungen und damit zu einer möglichen Zerstörung der Sonotrode selbst.

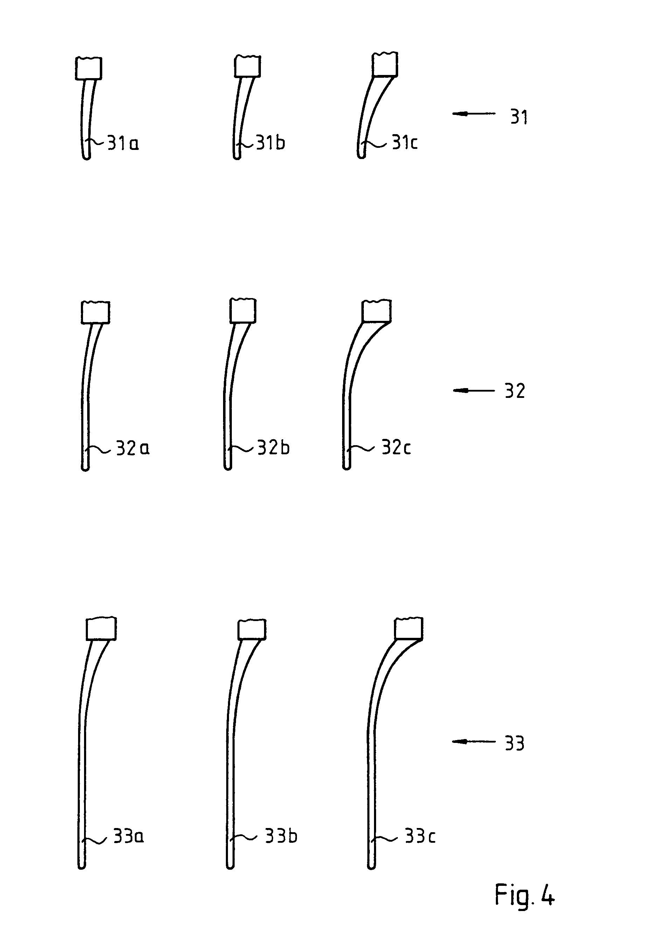

Um die Knochentzementröhre bei sämtlichen vorkommenden Prothesen entfernen zu können wurde ein Satz von Führungsabschnitten mit Sontroden geschaffen, wie er in Figur 4 dargestellt ist. In dargestellte Übersicht sind neun Varianten von Sonotrodenformen 31a, 31b, 31c, 32a, 32b, 32c, 33a, 33b und 33c vorgesehen. Diese lassen sich in drei Gruppen 31, 32 und 33 jeweils gleicher Länge aber abgestuft unterschiedlicher Krümmungen einteilen, wobei in der Darstellung von oben nach unten die Länge und von links nach rechts die Krümmung zunimmt. Die Längen- und die Krümmungsabstufungen sind dabei zweckmäßigerweise denjenigen der handelsüblichen, zur Knochenaushöhlung zu verwendenden Raspeln entlehnt.

Die Formen sind dabei so gewählt, daß bei Querschnittsabnahme zum freien Ende hin letzteres den tiefsten Bereich der Knochenröhre erreicht. Der Führungsabschnitt selbst ist relativ schlank gehalten, damit er jeweils für möglichst viele Prothesentypen der jeweiligen Längen- oder Krümmungsklasse verwendbar ist und weiterhin auch eine genügende Bewegungsfreiheit zur Verfügung steht um das die Sonotrode enthaltende Ende allen Teilen der Innenwandung der Knochenröhre anzunähern. Der aktive Sonotrodenteil selbst befindet sich jeweils bevorzugt im geraden Endbereich der dargesellten Formen der Führungabschnitte, da insoweit die Gefahr einer Beanspruchung der Sonotrode durch Biegeschwingungen minimiert ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Vorrichtung zum Entfernen einer nach dem Entfernen des Schafts einer einzementierten Endoprothese innerhalb der Knochenhöhlung verbleibenden Knochenzementröhre (5), wobei die Vorrichtung einen Ultraschallerzeuger (1) mit einer Sonotrode (3) aufweist,
**dadurch gekennzeichnet,**
daß der Ultraschallerzeuger (1) einen, insbesondere vollständig, in den Hohlraum der Knochenzementröhre (5) einführbaren und zur Führung des Ultraschallerzeugers (1) in diesem Hohlraum dienenden zylinder- oder schaftförmigen Führungsabschnitt (2) aufweist und
daß das freie, in den Hohlraum einführbare Ende des Führungsabschnittes (2) die ultraschallemittierende Sonotrode (3) bildet.

2. Vorrichtung nach Anspruch 1, **dadurch gekennzeichnet,** daß die Sonotrode (3) eine abgerundete Form aufweist.

3. Vorrichtung nach einem der vorangehenden Ansprüche, **dadurch gekennzeichnet,** daß die aktive Fläche der Sonotrode (3) durch die Mantelfläche eines koaxial zur Längsachse des Führungsabschnittes (2) ausgerichteten im wesentlichen zylindrischen Bereichs gebildet wird.

4. Vorrichtung nach einem der Ansprüche 1 bis 2, **dadurch gekennzeichnet,** daß ein Flächenvektor der aktiven Fläche der Sonotrode (3) radial zur Längsachse des Führungsabschnittes (2) gerichtet ist.

5. Vorrichtung nach einem der vorangehenden Ansprüche, **dadurch gekennzeichnet,** daß Sonotrode (3) aus einer Aluminium- oder Titanlegierung besteht.

6. Vorrichtung nach einem der vorangehenden Ansprüche, **dadurch gekennzeichnet,** daß die Länge der Sonotrode (3) und die Ultraschallfrequenz derart bemessen sind, daß die Länge der Sonotrode (3) im wesentlichen gleich einem Vielfachen der halben sich innerhalb der Sonotrode (3) einstellenden Wellenlänge ist.

7. Vorrichtung nach einem der vorangehenden Ansprüche, **gekennzeichnet durch** ein zusätzliches Führungsrohr (10 bzw. 24), dessen Innendurchmesser an den Außendurchmesser des Führungsabschnittes (2) angepaßt ist.

8. Vorrichtung nach Anspruch 7, **dadurch gekennzeichnet,** daß das Führungsrohr (24) mit einer Nut (16') und der Führungsabschnitt (2) mit einem in die Nut (16') eingreifenden Führungselement (17') versehen ist, wobei die Nut (16') eine Bewegungskurve der Sonotrode (3) in Bezug auf das Führungrohr (24) erzwingt, bei deren Durchlaufen ein geschlossener größerer Bereich der Knochenzementröhre (5) nacheinander in die Nachbarschaft des aktiven Bereichs der Sonotrode (3) gelangt.

9. Vorrichtung nach Anspruch 8, **gekennzeichnet durch** einen zusätzlichen Topfkurvenzylinder (12 bzw. 20), der auf dem Rand der Knochenzementröhre (5) aufsetzbar ist und in dessen Nut (13 bzw. 16) ein Nippel (17) des Führungsrohres (10) eingreift.

10. Vorrichtung nach einem der Ansprüche 7 bis 9, **dadurch gekennzeichnet,** daß das Führungsrohr (10 bzw. 24) weiterhin zum Durchlaß eines Endoskopes (27), einer Spülvorrichtung und/oder eines Spreizinstrumentes (25) ausgebildet ist.

11. Vorrichtung nach Anspruch 1, **dadurch gekennzeichnet,** daß der sich zu seinem freien Ende (30) hin verjüngende, die Sonotrode (3) aufweisende Führungsabschnitt (28 bzw. 29) einen dem Prothesenschaft angepaßten gekrümmten Bereich aufweist, wobei die Krümmung jeweils so gewählt ist, daß der einen konstanten Querschnitt aufweisende oder sich zu seinem freien Ende (30) hin gleichmäßig verjüngende Führungsabschnitt (28 bzw. 29) bei insgesamt möglichst geringer Krümmung den am weitesten von dem äußeren Zugang entfernten Bereich der Knochenzementröhre (5) erreicht und/oder der die Sonotrode (3) aufweisende Endbereich möglichst gestreckt ausgebildet ist.

12. Vorrichtung nach Anspruch 11, **dadurch gekennzeichnet,** daß mehrere auswechselbare Sonotroden aufweisende Führungsabschnitte (31, 32, 33) unterschiedlicher Länge und/oder unterschiedlicher Krümmung vorgesehen sind.

## Claims

1. A device for removing a bone cement tube (5) remaining inside the bone cavity after removal of the shaft of a cemented-in endoprosthesis, the device comprising an ultrasonic generator (1) with a sonotrode (3), characterized in that the ultrasonic generator (1) comprises a cylindrical or shaft-shaped guide portion (2) insertable, especially completely, into the hollow space in the bone cement tube (5) and serving to guide the ultrasonic generator (1) in this hollow space and in that the free end, insertable into the hollow space, of the guide portion (2) forms the ultrasonically emitting sonotrode (3).

2. A device according to claim 1, characterized in that the sonotrode (3) has a rounded shape.

3. A device according to either of the preceding claims, characterized in that the active area of the sonotrode (3) is formed by the outer surface of a substantially cylindrical area of coaxial orientation with the longitudinal axis of the guide portion (2).

4. A device according to either one of claims 1 and 2, characterized in that a planar vector of the active area of the sonotrode (3) is oriented radially with respect to the longitudinal axis of the guide portion (2).

5. A device according to any one of the preceding claims, characterized in that the sonotrode (3) consists of an aluminium or titanium alloy.

6. A device according to any one of the preceding claims, characterized in that the length of the sonotrode (3) and the ultrasonic frequency are so selected that the length of the sonotrode (3) is substantially equal to a multiple of the one-half wavelength arising inside the sonotrode (3).

7. A device according to any one of the preceding claims, characterized by an additional guide tube (10 or 24), whose internal diameter is conformed to the external diameter of the guide portion (2).

8. A device according to claim 7, characterized in that the guide tube (24) is provided with a groove (16') and the guide portion (2) is provided with a guide member (17') engaging in the groove (16'), the groove (16') forcing the sonotrode (3) to move in a curve with respect to the guide tube (24), as a result of moving through which one closed, relatively large area of the bone cement tube (5) after another comes into the vicinity of the active area of the sonotrode (3).

9. A device according to claim 8, characterized by an additional cup cam cylinder (12 or 20), which can be positioned on the edge of the bone cement tube (5) and in whose groove (13 or 16) there engages a nipple (17) of the guide tube (10).

10. A device according to any one of claims 7 to 9, characterized in that the guide tube (10 or 24) is further constructed for the passage of an endoscope (27), a flushing device and/or an expansion instrument (25).

11. A device according to claim 1, characterized in that the guide portion (28 or 29) tapering towards its free end (30) and comprising the sonotrode (3) comprises a curved area conformed to the prosthesis shaft, the curvature being such that the guide portion (28 or 29) comprising a constant cross-section or tapering uniformly towards its free end (30) reaches the area of the bone cement tube (5) furthest from the external access with the altogether slightest curvature possible and/or the end area comprising the sonotrode (3) is of the most extended construction possible.

12. A device according to clain 11, characterized in that a plurality of guide portions (31, 32, 33) of different lengths and/or different curvatures and comprising exchangeable sonotrodes are provided.

## Revendications

1. Dispositif pour enlever un tube de cimentation des os (5) restant à l'intérieur de l'évidement de l'os après le retrait de la tige d'une endoprothèse cimentée, le dispositif présentant un générateur d'ultra-sons (1) avec une sonotrode (3), caractérisé en ce que le générateur d'ultra-sons (1) présente une section de guidage (2) cylindrique ou en forme de tige pouvant être introduite, en particulier entièrement, dans l'évidement du tube de cimentation des os (5) et servant au guidage du générateur d'ultra-sons (1) dans cet évidement et en ce que l'extrémité libre, pouvant être introduite dans l'évidement, de la section de guidage (2) constitue la sonotrode (3) émettant les ultra-sons.

2. Dispositif selon la revendication 1, caractérisé en ce que la sonotrode (3) présente une forme arrondie.

3. Dispositif selon l'une ou l'ensemble des revendications précédentes, caractérisé en ce que la surface effective de la sonotrode (3) est formée par la surface d'enveloppe d'un secteur sensiblement cylindrique coaxial à l'axe longitudinal de la section de guidage (2).

4. Dispositif selon l'une ou l'ensemble des revendications 1 à 2, caractérisé en ce qu'un vecteur de surface de la surface active de la sonotrode (3) est orienté dans le sens radial par rapport à l'axe longitudinal de la section de guidage (2).

5. Dispositif selon l'une ou l'ensemble des revendications précédentes, caractérisé en ce que la sonotrode (3) se compose d'un alliage d'aluminium ou de titane.

6. Dispositif selon l'une ou l'ensemble des revendications précédentes, caractérisé en ce que la longueur de la sonotrode (3) et la fréquence des ultrasons sont choisies de telle sorte que la longueur de la sonotrode (3) soit sensiblement égale à un multiple de la moitié de la longueur d'onde s'établissant à l'intérieur de la sonotrode (3).

7. Dispositif selon l'une ou l'ensemble des revendications précédentes, caractérisé en ce qu'il comporte un tube de guidage (10 ou 24) supplémentaire, dont le diamètre intérieur est adapté au diamètre extérieur de la section de guidage (2).

8. Dispositif selon la revendication 7, caractérisé en ce que le tube de guidage (24) est pourvu d'une gorge (16') et la section de guidage (2) est pourvue d'un élément de guidage (17') se mettant en prise dans la gorge (16'), la gorge (16') contraignant à une courbe de déplacement de la sonotrode (3) par rapport au tube de guidage (24), sur le parcours de laquelle un grand secteur fermé du tube de cimentation des os (5) parvient successivement au voisinage du secteur actif de la sonotrode (3).

9. Dispositif selon la revendication 8, caractérisé en ce qu'il comporte un cylindre à courbe en creux (12 ou 20), qui peut être posé sur le bord du tube de cimentation des os (5) et dans la gorge (13 ou 16) duquel un ergot (17) du tube de guidage (10) se met en prise.

10. Dispositif selon l'une ou l'ensemble des revendications 7 à 9, caractérisé en ce que le tube de guidage (10 ou 24) est en outre conçu pour laisser passer un endoscope (27), un dispositif de lavage et/ou un instrument écarteur (25).

11. Dispositif selon la revendication 1, caractérisé en ce que la section de guidage (28 ou 29) se rétrécissant vers son extrémité libre (30) et présentant la sonotrode (3) présente un secteur recourbé adapté à la tige de prothèse, la courbure étant choisie de telle sorte que la section de guidage (28 ou 29) présentant une section constante ou se rétrécissant uniformément vers son extrémité libre (30) atteigne, avec une courbure globalement réduite, le secteur du tube de cimentation des os (5) le plus éloigné de l'accès extérieur et/ou que le secteur d'extrémité présentant la sonotrode (3) soit aussi allongé que possible.

12. Dispositif selon la revendication 11, caractérisé en ce que plusieurs sections de guidage (31, 32, 33) de longueurs différentes et/ou de courbures différentes présentant des sonotrodes interchangeables sont prévues.
